# EUROPÄISCHE PATENTANMELDUNG

(11) **EP 3 785 760 A1**
(43) Veröffentlichungstag der Anmeldung: **03.03.2021**
(21) Anmeldenummer: 20187393.2
(22) Anmeldetag: 23.07.2020
(51) Int. Cl.: A61N 1/36, H04R 25/00

(54) **VERFAHREN ZUM VERBESSERN DER HÖRQUALITÄT EINES MENSCHEN, COCHLEA-IMPLANTAT SOWIE COCHLEA-IMPLANTATSYSTEM**

(30) Priorität: 25.07.2019 US 201962878405 P
(71) Anmelder: Gottfried Wilhelm Leibniz Universität Hannover, 30167 Hannover (DE); Medizinische Hochschule Hannover, 30625 Hannover (DE)
(72) Erfinder: Ostermann, Jörn, 30655 Hannover (DE); Hinrichs, Reemt, 30167 Hannover (DE); Nogueira, Waldo, 30177 Hannover (DE); Preihs, Stephan, 30459 Hannover (DE)
(74) Vertreter: Weidner Stern Jeschke

(57) **Zusammenfassung**

Die Erfindung betrifft ein Verfahren zur Verbesserung der Hörqualtität eines Menschen mit Höreinschränkung sowie ein Cochlea-Implantat und ein Cochlea-Implantatsystem. Bekannte Verfahren und Cochlea-Implantate weisen den Nachteil auf, dass der Austausch von Daten und Informationen zwischen unterschiedlichen Bauteilen mit relativ hoher Datenrate erfolgen muss und damit ein hoher Verbrauch von elektrischer Energie einhergeht. Es wird ein Verfahren zum Verbessern der Hörqualität eines Menschen mit Höreinschränkung mittels eines Cochlea-Implantats vorgeschlagen, wobei das Cochlea-Implantat eine dem Cochlea-Implantat zugeordnete externe Einheit aufweist und das Cochlea-Implantat mittels einer Elektrode oder mehrerer Elektroden mit dem Hörapparat des Menschen wirksam verbunden ist und das Cochlea-Implantat und die externe Einheit zur Aufnahme von akustischen Schallsignalen eingerichtet sind.

## Beschreibung

Die Erfindung betrifft ein Verfahren zum Verbessern der Hörqualität eines Menschen mit Höreinschränkung mittels eines Cochlea-Implantats, ein Cochlea-Implantat sowie ein Cochlea-Implantatsystem.

Cochlea-Implantate werden dazu verwendet, Menschen mit Höreinschränkung oder Menschen ohne Hörvermögen dennoch einen Höreindruck zu vermitteln. Dazu weisen Cochlea-Implantate Einrichtungen zur Aufnahme von Schallsignalen auf. Zudem weisen Cochlea-Implantate Bestandteile auf, welche diese Schallsignale umwandeln und in elektronischer Form an Teile des Hörapparates eines Menschen weiterleiten, zum Beispiel an die Hörschnecke (Cochlea). Dies geschieht mit invasiv installierten Elektroden am Hörapparat.

Bei bekannten Verfahren zum Betrieb solcher Cochlea-Implantate werden akustische Schallsignale sowohl vom Cochlea-Implantat als auch weiteren, dem Cochlea-Implantat zugeordneten, Bauteilen aufgenommen und aus diesen Signalen ein vergleichsweise räumlicher Höreindruck generiert. Dies führt beispielsweise zur Verbesserung des Sprachverständnisses oder des Raumhörvermögens eines Trägers eines Cochlea-Implantats.

Aus der EP 2 92 976 B1 ist ein Tonverbesserungsverfahren für Cochlea-Implantat-Tonprozessoren bekannt, wobei eingehende Tonsignale erfasst, aufgeteilt und dann Hüllkurven für jedes Frequenzband erzeugt, komprimiert und übertragen werden.

Die US 10,342,976 B2 offenbart ein Cochlea-Implantatsystem mit Mikrofonen zur Aufnahme von Audiosignalen und einem selektiven Geräuschprozessor.

In der US 6,480,820 B1 wird ein Verfahren zur Echtzeit-Umwandlung eines elektrischen Signals einer akustischen Information beschrieben. Es werden unter anderem Bandpassfilter verwendet.

Bei der Durchführung solcher Verfahren ist es nachteilig, dass teilweise große Datenmengen zwischen Bestandteilen des Cochlea-Implantats übertragen werden müssen, wobei diese Übertragung zumeist per Funk erfolgt. Insbesondere in Bezug auf die Batterieleistung stellt dies einen Nachteil dar, da ein solches Cochlea-Implantat dann geringe Betriebszeiten aufweist. Weiterhin kann es bei bekannten Verfahren zu Verzögerungen in der Signalverarbeitung kommen, welche nachteilige akustische Effekte zur Folge haben.

Aufgabe der Erfindung ist es, den Stand der Technik zu verbessern.

Gelöst wird die Aufgabe durch ein Verfahren zum Verbessern der Hörqualität eines Menschen mit Höreinschränkung mittels eines Cochlea-Implantats, wobei das Cochlea-Implantat eine dem Cochlea-Implantat zugeordnete externe Einheit aufweist und das Cochlea-Implantat mittels einer Elektrode oder mehrerer Elektroden mit dem Hörapparat des Menschen wirksam verbunden ist und das Cochlea-Implantat und die externe Einheit zur Aufnahme von akustischen Schallsignalen eingerichtet sind und das Verfahren folgende Schritte umfasst:
- Aufnehmen von akustischen Schallsignalen mittels des Cochlea-Implantats, so dass erste elektronische Schallsignale vorliegen,
- Aufnehmen von akustischen Schallsignalen mittels der zweiten externen Einheit, so dass zweite elektronische Schallsignale vorliegen,
- Erzeugen eines Elektrodogramms der zweiten elektronischen Schallsignale, so dass ein Elektrodogramm vorliegt,
- Komprimieren des Elektrodogramms, so dass ein komprimiertes Elektrodogramm der akustischen Schallsignale vorliegt,
- Übertragen des komprimierten Elektrodogramms der akustischen Schallsignale an das Cochlea-Implantat,
- Dekomprimieren des Elektrodogramms, so dass ein dekomprimiertes Elektrodogramm vorliegt,
- Einbinden des dekomprimierten Elektrodogramms in die ersten elektronischen Schallsignale des Cochlea-Implantats, so dass kombinierte elektronische Schallsignale vorliegen, welche geeignet sind, dem Menschen einen Höreindruck der akustischen Schallsignale zu vermitteln,
- Einbringen der kombinierten elektronischen Schallsignale zur Stimulation des Hörapparates mittels der Elektrode oder der Elektroden in den Hörapparat des Menschen,
so dass mittels des Cochlea-Implantats eine verbesserte Hörqualität für den Menschen erreicht wird.

Damit kann das Übertragen der Informationen von der externen Einheit an das Cochlea-Implantat besonders einfach und mit geringer Datenrate erfolgen. Die Verwendung eines Elektrodogramms als Informationsträger für diese Übertragung stellt eine besonders geringe Datenmenge sicher. Dabei können auch schon für das Übertragen nicht relevante Inhalte ausgeblendet oder entfernt sein, beispielsweise solche Informationen von Elektroden, welche keine Informationen für eine Verbesserung der Hörqualität enthalten. Dennoch enthält das Elektrodogramm alle für den Träger eines Cochlea-Implantats notwendigen Informationen des akustischen Schallsignals. Insbesondere ist durch das Komprimieren und Dekomprimieren des Elektrodogramms gegenüber dem Komprimieren einer rein akustischen Information ein deutlicher Zeitvorteil während dem Übertragen erzielbar, so dass keine laufzeitbedingten Echos oder andere Störungen den Träger eines solchen Cochlea-Implantats nachteilig beeinflussen oder den Höreindruck negativ beeinflussen.

Folgende Begriffe seien an dieser Stelle erläutert:
Die "Hörqualität" beschreibt die beispielsweise subjektiv oder auch objektiv wahrnehmbare oder messbare Wahrnehmung von Geräuschen aus der Umwelt. Dies kann sowohl Sprache als auch eine Summe aus Umgebungsgeräuschen sein, welche durch einen Menschen aufgenommen werden, um sich, beispielsweise, im Raum zu orientieren. Eine verbesserte Hörqualität kann also auch eine verbesserte akustische Orientierung im Raum sein. Weiterhin kann insbesondere bei Trägern von Cochlea-Implantaten die Verbesserung des Sprachverständnisses oder der Worterkennung als eine Verbesserung der Hörqualität verstanden werden.

Eine "Höreinschränkung" kann jede Beeinträchtigung der Wahrnehmung von Sprache oder Umgebungsgeräuschen durch einen Menschen sein. Insbesondere kann dies eine Einschränkung derart sein, dass der Mensch Sprache nicht mehr vollständig oder korrekt verstehen kann oder sich in einem Raum mittels der dort vorhandenen Umgebungsgeräusche nicht mehr zuverlässig orientieren kann. Auch kann die Worterkennung vermindert sein oder die Trennung von Gesprächen mit unterschiedlichen Personen erschwert sein.

Ein "Cochlea-Implantat" kann jede medizinischtechnische Einrichtung sein, welche einen Menschen beim Hören unterstützt, und zwar derart, dass Geräusche und/oder Sprache aus der Umgebung aufgenommen, bedarfsweise verstärkt und/oder umgewandelt und dem Menschen auf elektronische Weise zugeleitet werden, sodass sein Höreindruck oder die Hörqualität sich verbessern. Beispielsweise ist ein Cochlea-Implantat eine zusammenhängende technische Einrichtung, an der ein Mikrofon am Kopf eines Menschen externe Geräusche und/oder Sprache aufnimmt, umwandelt und mittels einer Übertragungsstelle an ein im Hörapparat des Menschen angebrachtes Implantat übertragt, und dort mittels elektronischer Signale auf den Hörapparat des Menschen einwirkt. Als Übertragungsstelle an den Hörapparat kann dabei die Hörschnecke (Cochlea) dienen.

Eine "externe Einheit" ist beispielsweise jede dem Cochlea-Implantat zugeordnete technische Einrichtung, mittels derer Schallsignale, wie zum Beispiel Sprache und/oder Umgebungsgeräusche aufgenommen werden können. Weiterhin können diese Signale mittels der externen Einheit oder in der externen Einheit umgewandelt und dem Cochlea-Implantat zugeleitet werden, zum Beispiel mittels eines Funksignals oder mittels elektromagnetischer Kopplung oder auf andere Weise.

Eine "Elektrode" kann jede, zum Beispiel drahtförmige, Einheit sein, welche geeignet ist, elektrische Signale weiterzuleiten und an entsprechender Stelle abzugeben oder umzuwandeln.

Der "Hörapparat eines Menschen" kann jedes Teil der Anatomie eines Menschen umfassen, welcher notwendig oder geeignet ist, Schallsignale oder Schallinformationen, wie zum Beispiel Sprache, Geräusche oder elektronische Repräsentanzen dieser Signale, aufzunehmen, weiterzuleiten und weiterzuverarbeiten und dem Menschen zuzuleiten. Dies kann beispielsweise das Ohr, das Innenohr, Knochen oder Knorpel sowie weitere Bestandteile sein, welche dem Ohr des Menschen zugeordnet sind und dazu dienen können, Schall zu verstehen. Ein Teil dieses Hörapparates ist auch die Hörschnecke.

Ein "Elektrodogramm" kann jede Darstellung oder elektronische Abbildung oder Repräsentanz von akustischen Signalen oder Darstellung oder elektronische Abbildung oder Repräsentanz von akustischen Signalen mit einer zusätzlichen Information sein. [Dieses zusätzliche Information kann Name Nr. Elektrode] Beispielsweise ist ein Elektrodogramm eine zweidimensionale elektronische Repräsentanz eines Stimulationsmusters eines Cochlea-Implantats für ein definiertes Schallsignal, insbesondere getrennt nach unterschiedlichen Kanälen oder Elektroden. Eine erste Achse oder eine erste Dimension kann oder können dabei die Zeit darstellen, eine zweite Achse oder zweite Dimension kann oder können die Frequenz abbilden. Eine zusätzliche Codierung an einer jeweiligen Position im Elektrodogramm kann die Intensität oder Leistungsdichte oder Energiedichte des jeweiligen Schallsignals mit der jeweiligen Frequenz zum abgebildeten Zeitpunkt repräsentieren. Damit repräsentiert das Elektrodogramm das akustische Schallsignal gemäß Amplitude und Frequenz und kann dies beispielsweise in elektronischer Form bereitstellen. Ein Elektrodogramm kann auch die von einer jeweiligen Elektrode oder mehreren Elektroden erzeugte Spannung oder den von einer jeweiligen Elektrode oder mehreren Elektroden erzeugten Stromfluss elektronisch abbilden. Ein solches Elektrodogramm kann dann in elektronischer Form weiterverarbeitet oder auch graphisch angezeigt werden.

Ein "kombiniertes elektronisches Schallsignal" kann jede elektronisch vorliegende oder verarbeitungsfähige Information zu akustischen Schallsignalen sein, welche aus den ersten elektronischen Schallsignalen und beispielsweise dem dekomprimierten Elektrodogramm gebildet werden. Die Kombination kann dabei nach mathematischen Methoden, gemäß einer Algorithmik oder nach medizinischen Gesichtspunkten erfolgen, so dass im Ergebnis kombinierte elektronische Schallsignale vorliegen, welche geeignet sind, dem Menschen einen Höreindruck der akustischen Schallsignale zu vermitteln, der insbesondere das Sprachverständnis oder das Raumhörvermögen verbessert. Beispielsweise kann dies auch jede Operation oder Handlung mit oder an Schallsignalen sein, welche Informationen aus mehreren Elektrodogrammen extrahiert und daraus ein neues Elektrodogramm erzeugt oder eine Signalverarbeitungsstrategie des Cochlea-Implantats oder mehrerer Cochlea-Implantate entsprechend der gewonnenen Information anpasst.

In einer Ausführungsform ist die externe Einheit ein zweites Cochlea-Implantat.

Damit kann das zweite Cochlea-Implantat mit dem Cochlea-Implantat verbunden sein und so einseitig oder wechselseitig mit dem erfindungsgemäßen Verfahren dazu beitragen, dass der Höreindruck oder die Hörqualität des Menschen verbessert wird. Weiterhin kann bei beidseitiger Hörbeeinträchtigung das zweite, für den Menschen dann notwendige, Cochlea-Implantat ohne weitere Bauteile genutzt werden, um die Hörqualität für den Menschen zu verbessern, insbesondere das Raumhörvermögen. Das Dekomprimieren des Elektrodogramms kann dann z.B. auch im Cochlea-Implantat erfolgen.

Um das Cochlea-Implantat oder das zweite Cochlea-Implantat komfortabel einzurichten und während der Benutzung beispielsweise das Nachladen des Cochlea-Implantats mit elektrischer Energie einfach zu gestalten, weist das Cochlea-Implantat oder das zweite Cochlea-Implantat eine externe Einheit und eine interne Einheit auf, wobei die externe Einheit zur Aufnahme von akustischen Signalen eingerichtet ist und die interne Einheit die Elektrode oder die Elektroden umfasst.

In einer Ausführungsform wird das Elektrodogramm mittels der Statistical Dependency Removing Coding Technique, insbesondere einer DPCM (Differential Pulse Code Modulation) komprimiert. Dies kann auch mit einer verlustlosen Kompression einer Bandselektion kombiniert sein.

Ein Komprimieren mit dieser Methode oder der Kombination der Methoden stellt sicher, dass die Datenmenge zum Übertragen des Elektrodogramms möglichst geringgehalten wird. Damit muss dann auch nur wenig elektrische Energie zum Übertragen aufgewendet werden, wodurch die Laufzeit eines Cochlea-Implantats zwischen dem Aufladen von, zum Beispiel, integrierten Akkumulatoren, deutlich verlängert wird.

In einer weiteren Ausführungsform kann das Elektrodogramm auch mittels Transformationscodierungen oder Vektorquantisierung komprimiert werden.

Die "DPCM" ist ein Kompressionsverfahren, mit beispielsweise bei gleichem Kompressionsgrad die Qualität im Vergleich zu einer direkten Quantisierung verbessert wird. Es kann beispielsweise eine Varianz eines Prädiktionsfehlers eines Differenzsignals zwischen einem komprimierten Signal und einem dekomprimierten Signal verringert werden kann. Um diese Varianz zu verringern, kann in prädiktiven oder differentiellen Codierungen das Signal, welches komprimiert werden soll, vorhergesagt und anschließend ein Prädiktionsfehler, also eine Differenz zwischen der Prädiktion und einem Ursprungssignal, quantisiert werden. Während der Dekompression kann die Prädiktion auf den Prädiktionsfehler addiert werden, so dass Näherungsweise das Originalsignal erzeugt werden kann.

Dabei kann die DPCM zur Klasse der differentiellen und prädiktiven Codierungsverfahren gehören und auch derart ausgeführt werden, dass der Prädiktor adaptiv ist, die Quantisierung jedoch fest. Alternativ kann die DPCM auch so ausgeführt werden, dass der Prädiktor und der Quantisierer fest gewählt sind. Eine adaptive DPCM (ADPCM) kann eine DPCM sein, bei welchem ein Prädiktor und/oder ein Quantisierer angepasst werden, beispielsweise in einem Zeitintervall oder einer Taktfolge.

Um die Datenrate weiter zu minimieren, kann noch eine verlustlose Codierung, beispielsweise eine Entropiecodierung, verwendet werden, welcher beispielsweise bei DPCMs mit statischen Quantisierern verwendet wird.

Um noch weniger Daten übertragen zu müssen, wird eine Komprimierung des Elektrodogramms des jeweiligen Schallsignals mittels eines ACE (Advanced Combination Encoder) angewandt oder durch einen ACE unterstützt, insbesondere mittels eines Psychoacoustic Advanced Combination Encoder. Dabei wird eine Anzahl N < M Frequenzbänder aus einem Frequenzspektrum des Schallsignals derart ausgewählt, dass nur eine solche Anzahl N Frequenzbänder aus der Anzahl M der gesamten Frequenzbänder übertragen werden, welche einer Amplitude oberhalb einer Hörschwelle des Menschen aufweisen.

Die "ACE" kann dabei solche als relevant erachteten Kanäle oder Signalwege auswählen und nur deren Signale übertragen. So kann mittels der ACE beispielsweise aus einer Gesamtheit von 22 Elektroden eines Cochlea-Implantats eine Zahl von 8 relevanten Elektroden ausgewählt und nur deren Signale übertragen werden. Es ist aber auch jede andere Anzahl von ausgewählten Elektroden möglich.

Die "Hörschwelle des Menschen" ist dabei beispielsweise die, je Mensch individuelle, Lautstärke von Schallsignalen, welche als minimale Lautstärke noch wahrgenommen werden kann.

In einer Ausführungsform werden von den akustischen Schallsignalen nur solche Schallinformationen in das Elektrodogramm umgewandelt, welche einen üblicherweise von einem Menschen wahrnehmbaren Frequenzbereich abbilden, insbesondere einer Frequenz zwischen 20 Hz und 20 kHz. Beispielsweise kann dabei ein Frequenzbereich gewählt werden, welcher zwischen 8kHz und 10kHz liegt.

Damit werden nur solche Schallinformationen übertragen, welche von einem Menschen, insbesondere einem Träger eines Cochlea-Implantats, auch tatsächlich wahrgenommen werden können. Diese Verfahrensweise reduziert zusätzlich den notwendigen Datenstrom und verlängert damit auch die Laufzeit des Cochlea-Implantats.

Um eine noch geringere Datenmenge übertragen zu müssen, wird oder werden ein Erregungsschema oder mehrere Erregungsschemata für eine Elektrode oder für mehrere Elektroden oder für alle Elektroden mittels einer DPCM (Differential Pulse-Code Modulation) komprimiert, wobei daraus abgeleitete Quantisierungsindizes dieser Erregungsschemata verlustfrei komprimiert werden.

In einer Ausführungsform weist das übertragene Elektrodogramm eine Information über eine vorliegende oder fehlende Veränderung zu einem vorher übertragenen Elektrodogramm auf.

Mittels dieser Information kann das jeweilige Elektrodogramm so gekennzeichnet werden, dass eine vorliegende oder fehlende Veränderung zu einem vorher übertragenen Elektrodogramm nach der Übertragung sicher erkannt werden kann.

Um mittels dieser Information die zu übertragende Datenmenge noch weiter zu reduzieren, erfolgt nur bei vorliegen einer Veränderung zum vorher übertragenen Elektrodogramm eine Übertragung des komprimierten Elektrodogramms. Weiterhin kann nur dann die Übertragung von einer veränderten Bandselektion oder einer veränderten Elektrodenauswahl erfolgen, wenn diese eine Veränderung zu einer vorher übertragenen Bandselektion oder vorher übertragenen Elektrodenauswahl aufweist.

In einer Ausführungsform erfolgt die Übertragung des komprimierten Elektrodogramms drahtlos, insbesondere mittels einer Funkstrecke oder einer Near-Field Magnetic Induction oder per Bluetooth.

Damit können weitere technische Einrichtungen eingespart und eine sichere Übertragung gewährleistet werden. Weiterhin kann die Übertragung mit bekannten, zuverlässigen und stromsparenden Methoden erfolgen. Insbesondere per Bluetooth ist dabei eine Einkopplung auch in externe Geräte, wie zum Beispiel Mobiltelefone, möglich. Die Verwendung der Near-Field Magnetic Induction stellt sicher, dass eine Übertragung durch menschliches Gewebe, z.B. den Kopf, auf direktem Wege mit wenig Beeinträchtigung durch das menschliche Gewebe erfolgt.

In einem weiteren Aspekt wird die Aufgabe gelöst durch ein Cochlea-Implantat für einen Menschen mit Höreinschränkung, wobei das Cochlea-Implantat eine dem Cochlea-Implantat zugeordnete externe Einheit aufweist und mittels einer Elektrode oder mehrerer Elektroden mit dem Hörapparat des Menschen wirksam verbunden ist und das Cochlea-Implantat und die externe Einheit zur Aufnahme von akustischen Schallsignalen eingerichtet sind, wobei das Cochlea-Implantat derart eingerichtet ist, dass ein Verfahren gemäß einer oder mehrerer der genannten Ausführungsformen durchführbar ist.

Ein solches Cochlea-Implantat kann alle Vorteile der oben genannten Ausgestaltungen des erfindungsgemäßen Verfahrens nutzen und so besonders energiesparend und akustisch wirkungsvoll betrieben werden.

In einem abschließenden Aspekt wird die Aufgabe gelöst durch ein Cochlea-Implantatsystem für einen Menschen, bestehend aus einem Cochlea-Implantat oder mehreren Cochlea-Implantaten, welche derart eingerichtet ist oder sind, dass ein Verfahren nach einem der oben genannten Ausführungsformen durchführbar ist.

Ein solches Cochlea-Implantatsystem kann alle oder mehrere genannten Vorteile des erfindungsgemäßen Verfahrens nutzen und umsetzen, sodass die Hörqualität des Menschen verbessert wird.

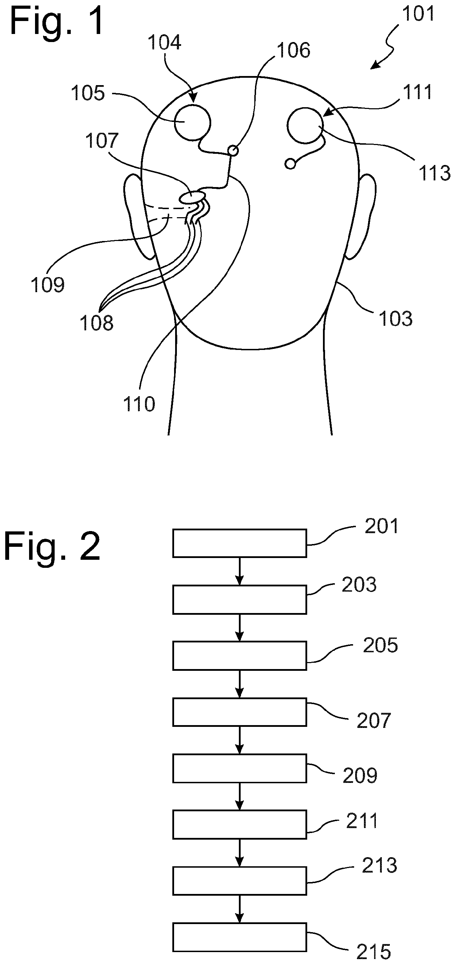

Im Weiteren wird die Erfindung anhand von Ausführungsbeispielen erläutert. Es zeigen
- Figur 1: eine schematische Darstellung eines Cochlea-Implantatsystems, welches mit dem erfindungsgemäßen Verfahren betrieben wird, sowie
- Figur 2: ein Flussdiagramm eines Verfahrens zum Verbessern der Hörqualität eines Menschen.

Ein Cochlea-Implantatsystem 101 ist beispielshaft an einem Kopf 103 angebracht. Der Kopf 103 ist ein Kopf eines höreingeschränkten Menschen. Das Cochlea-Implantatsystem 101 besteht aus einem ersten Cochlea-Implantat 104 sowie einem zweiten Cochlea-Implantat 111. Das erste Cochlea-Implantat 104 ist dabei schematisch vollständig dargestellt, das zweite Cochlea-Implantat 111 nur mit der außerhalb des Kopfes 103 liegenden externen Einheit 113.

Das erste Cochlea-Implantat 104 besteht aus einer externen Einheit 105, welche mit einem Kontaktpunkt 106 an eine interne Einheit 107 angebunden ist. Dazu ist eine Verbindung 110 vorhanden. Das erste Cochlea-Implantat 104 sowie das zweite Cochlea-Implantat 111 wurden mit einem invasiven Eingriff in den Kopf 103 eingesetzt, um dem Menschen einen Höreindruck durch zum Beispiel ein besseres Raumhörvermögen zu ermöglichen.

An der Verbindung 110 ist die interne Einheit 107 angeschlossen. Von der internen Einheit 107 führen Elektroden 108 zum Hörapparat 109 des Menschen. Die Elektroden 108 sind dabei bei der Installation des ersten Cochlea-Implantats 104 an den Hörapparat 109, nämlich die Hörschnecke, derart angeschlossen, dass über die Elektroden 108 elektronische Signale in den Hörapparat 109 eingeleitet werden können. Mittels dieser elektronischen Signale nimmt der Mensch dann im Kopf 103 den Höreindruck wahr.

Das zweite Cochlea-Implantat 111 steht mit dem ersten Cochlea-Implantat 104 über eine Funkverbindung in Kontakt. Weiterhin sind im ersten Cochlea-Implantat 104 in der externen Einheit 105 sowie analog dazu im zweiten Cochlea-Implantat 111 ebenfalls in der dargestellten externen Einheit jeweils ein Mikrofon vorhanden, wobei die Mikrofone akustische Signale von außerhalb des Kopfes 103 aufnehmen können. Das erste Cochlea-Implantat 104 sowie das zweite Cochlea-Implantat 111 haben zudem jeweils einen integrierten Akkumulator, der die Cochlea-Implantate 104 und 111 jeweils mit elektrischem Strom für deren Betrieb versorgt.

Im Folgenden soll der Betrieb des Cochlea-Implantatsystems 101 näher erläutert werden:

Befindet sich der Mensch in einer Umgebung, in der zum Beispiel Sprache oder andere Geräusche vorhanden sind, so nehmen die externe Einheit 105 des ersten Cochlea-Implantats 104 sowie das zweite Cochlea-Implantat 111 mit seiner externen Einheit 113 diese akustischen Signale auf. Im zweiten Cochlea-Implantat 111 werden diese akustischen Signale dann in ein Elektrodogramm verwandelt, damit liegen die zum akustischen Schallsignal gehörenden Informationen in Form des Elektrodogramms vor. Dieses Elektrodogramm wird dann in der externen Einheit 113 komprimiert, und zwar in diesem Beispiel mit der DPCM-Methode.

Sodann wird das komprimierte Elektrodogramm vom zweiten Cochlea-Implantat 111 an die externe Einheit 105 des ersten Cochlea-Implantats 104 übertragen. Innerhalb der externen Einheit 105 des ersten Cochlea-Implantats 104 wird dann das komprimierte Elektrodogramm dekomprimiert. Das dekomprimierte Elektrodogramm wird dann innerhalb der externen Einheit 105 mit den von der externen Einheit 105 aufgenommenen akustischen Signalen so überlagert, dass der von beiden Cochlea-Implantaten 104 sowie 111 aufgenommene, räumliche Höreindruck in einem elektronischen Signal vorliegt.

Das elektronische, aus beiden Cochlea-Implantaten 104 und 111 überlagerte elektronische Signal wird dann über den Kontaktpunkt 106 an die Verbindung 110 und von dort weiter an die interne Einheit 107 übertragen.

In der internen Einheit 107 werden die elektronischen Signale derart aufbereitet, dass sie mittels der Elektroden 108 in den Hörapparat 109 eingebracht werden. Durch die Einbringung dieser elektronischen Signale mittels der Elektroden 108 in den Hörapparat 109 kann dann im Kopf 103 des Menschen ein verbesserter räumlicher Höreindruck entstehen.

Im Weiteren werden die Verfahrensschritte dargestellt.

Im ersten Cochlea-Implantat 104 erfolgt ein Aufnehmen 201 von akustischen Schallsignalen. Sodann erfolgt im zweiten Cochlea-Implantat 111 ein Aufnehmen 203 von akustischen Schallsignalen mittels der zweiten externen Einheit 113.

Innerhalb des zweiten Cochlea-Implantats 111 wird dann ein Elektrodogramm erzeugt. Das Elektrodogramm wird innerhalb des zweiten Cochlea-Implantats 111 komprimiert 207.

Nach diesem Schritt erfolgt ein Übertragen 209 des komprimierten Elektrodogramms der akustischen Schallsignale an das erste Cochlea-Implantat 104. Innerhalb des ersten Cochlea-Implantats 104 erfolgt dann ein Dekomprimieren 211 des Elektrodogramms. Sodann erfolgt ein Einbinden 213 des dekomprimierten Elektrodogramms in die ersten elektronischen Schallsignale des Cochlea-Implantats 104. Abschließend werden dann die kombinierten Elektronischen Schallsignale zur Stimulation des Hörapparates mittels der Elektrode oder der Elektroden in den Hörapparat 109 eingebracht 215, so dass mittels des Cochlea-Implantatsystems 101 eine verbesserte Hörqualität für den Menschen erreicht wird.

### Bezugszeichenliste

101 Cochlea-Implantatsytem
103 Kopf
104 erstes Cochlea-Implantat
105 externe Einheit
106 Kontaktpunkt
107 interne Einheit
108 Elektrode
109 Hörapparat
110 Verbindung
111 zweites Cochlea-Implantat
113 Externe Einheit
201 Aufnehmen von Schallsignalen am ersten Cochlea-Implantat
203 Aufnehmen von Schallsignalen am zweiten Cochlea-Implantat
205 Erzeugen eines Elektrodogramms
207 Komprimieren des Elektrodogramms
209 Übertragen des komprimierten Elektrodogramms
211 Dekomprimieren des komprimierten Elektrodogramms
213 Einbinden des dekomprimierten Elektrodogramms
215 Einbringen in den Hörapparat

## Patentansprüche

1. Verfahren zum Verbessern der Hörqualität eines Menschen mit Höreinschränkung mittels eines Cochlea-Implantats (104), wobei das Cochlea-Implantat (104) eine dem Cochlea-Implantat (104) zugeordnete externe Einheit (111) aufweist und das Cochlea-Implantat (104) mittels einer Elektrode (108) oder mehrerer Elektroden (108) mit dem Hörapparat (109) des Menschen wirksam verbunden ist und das Cochlea-Implantat (104) und die externe Einheit (111, 113) zur Aufnahme von akustischen Schallsignalen eingerichtet sind und das Verfahren folgende Schritte umfasst:
- Aufnehmen (201) von akustischen Schallsignalen mittels des Cochlea-Implantats (104), so dass erste elektronische Schallsignale vorliegen,
- Aufnehmen (203) von akustischen Schallsignalen mittels der zweiten externen Einheit (113), so dass zweite elektronische Schallsignale vorliegen,
- Erzeugen (205) eines Elektrodogramms der zweiten elektronischen Schallsignale, so dass ein Elektrodogramm vorliegt,
- Komprimieren (207) des Elektrodogramms, so dass ein komprimiertes Elektrodogramm der akustischen Schallsignale vorliegt,
- Übertragen (209) des komprimierten Elektrodogramms der akustischen Schallsignale an das Cochlea-Implantat (104),
- Dekomprimieren (211) des Elektrodogramms, so dass ein dekomprimiertes Elektrodogramm vorliegt,
- Einbinden (213) des dekomprimierten Elektrodogramms in die ersten elektronischen Schallsignale des Cochlea-Implantats (104), so dass kombinierte elektronische Schallsignale vorliegen, welche geeignet sind, dem Menschen einen Höreindruck der akustischen Schallsignale zu vermitteln,
- Einbringen (205) der kombinierten Elektronischen Schallsignale zur Stimulation des Hörapparates (109) mittels der Elektrode (108) oder der Elektroden (108) in den Hörapparat (109) des Menschen,
so dass mittels des Cochlea-Implantats (104) eine verbesserte Hörqualität für den Menschen erreicht wird.

2. Verfahren gemäß Anspruch 1, **dadurch gekennzeichnet, dass** die externe Einheit ein zweites Cochlea-Implantat (111) ist.

3. Verfahren gemäß Anspruch 1 oder 2, **dadurch gekennzeichnet, dass** das das Cochlea-Implantat (104) oder das zweite Cochlea-Implantat (111) eine externe Einheit (105, 113) und eine interne Einheit (107) aufweisen, wobei die externe Einheit (105, 113) zur Aufnahme von akustischen Signalen eingerichtet ist und die interne Einheit (107) die Elektrode (108) oder die Elektroden (108) umfasst.

4. Verfahren gemäß einem der vorherigen Ansprüche, **dadurch gekennzeichnet, dass** das Elektrodogramm mittels der statistical dependency removing coding technique, insbesondere einer DPCM (differential pulse code modulation) komprimiert wird.

5. Verfahren gemäß einem der vorherigen Ansprüche, **gekennzeichnet durch** eine Komprimierung des Elektrodogramms des jeweiligen Schallsignals mittels eines ACE (advanced combination encoder), insbesondere einem psychoacoustic advanced combination encoder, wobei eine Anzahl N<M Frequenzbänder aus einem Frequenzspektrum des Schallsignals derart ausgewählt werden, dass nur eine solche Anzahl N Frequenzbänder aus der Anzahl M der gesamten Frequenzbänder übertragen werden, welche eine Amplitude oberhalb einer Hörschwelle des Menschen aufweisen.

6. Verfahren gemäß einem der vorherigen Ansprüche, **dadurch gekennzeichnet, dass** von den akustischen Schallsignalen nur solche Schallinformationen in das Elektrodogramm umgewandelt werden, welche einen üblicherweise von einem Menschen wahrnehmbaren Frequenzbereich abbilden, vorzugsweise einer Frequenz zwischen 20 Hz und 20 kHz oder einer Frequenz zwischen 8 kHz und 10 kHz.

7. Verfahren gemäß einem der vorherigen Ansprüche, **dadurch gekennzeichnet, dass** ein Erregungsschema oder mehrere Erregungsschemata für eine Elektrode oder für mehrere Elektroden oder für alle Elektroden mittels einer DPCM (differential pulse-code modulation) komprimiert wird oder werden, wobei daraus abgeleitete Summenindizes dieser Erregungsschemata verlustfrei komprimiert werden.

8. Verfahren gemäß einem der vorherigen Ansprüche, **dadurch gekennzeichnet, dass** das übertragene Elektrodogramm eine Information über eine vorliegende oder fehlende Veränderung zu einem vorher übertragenen Elektrodogramm aufweist.

9. Verfahren gemäß Anspruch 8, **dadurch gekennzeichnet, dass** nur bei Vorliegen einer Veränderung zum vorher übertragenen Elektrodogramm eine Übertragung des komprimierten Elektrodogramms erfolgt oder nur bei Vorliegen einer veränderten Bandselektion oder einer veränderten Elektrodenauswahl eine Übertragung der veränderten Bandselektion oder der veränderten Elektrodenauswahl erfolgt.

10. Verfahren gemäß einem der vorherigen Ansprüche, **dadurch gekennzeichnet, dass** die Übertragung des komprimierten Elektrodogramms drahtlos erfolgt, insbesondere mittels einer Funkstrecke oder Near-Field Magnetic Induction oder mittels Bluetooth.

11. Cochlea-Implantat (104) für einen Menschen mit Höreinschränkung, wobei das Cochlea-Implantat (104) eine dem Cochlea-Implantat (104) zugeordnete externe Einheit (111, 113) aufweist und mittels einer Elektrode (108) oder mehrerer Elektroden (108) mit dem Hörapparat (109) des Menschen wirksam verbunden ist und das Cochlea-Implantat (104) und die externe Einheit (111, 113) zur Aufnahme von akustischen Schallsignalen eingerichtet sind, **dadurch gekennzeichnet, dass** das Cochlea-Implantat (104) derart eingerichtet ist, dass ein Verfahren nach einem der Ansprüche 1 bis 10 durchführbar ist.

12. Cochlea-Implantatsystem (101) für einen Menschen bestehend aus einem oder mehreren Cochlea-Implantaten (104, 111), welche derart eingerichtet ist oder sind, dass ein Verfahren nach einem der Ansprüche 1 bis 10 durchführbar ist.
